(19) Europäisches Patentamt
European Patent Office
Office européen des brevets

(11) Publication number: 0 280 867 A1

# (12) EUROPEAN PATENT APPLICATION

(21) Application number: 88101024.3

(51) Int. Cl.⁴: C07D 249/12

(22) Date of filing: 25.01.88

(30) Priority: 27.01.87 US 7306
19.08.87 US 87018

(43) Date of publication of application:
07.09.88 Bulletin 88/36

(84) Designated Contracting States:
AT BE CH DE ES FR GB GR IT LI LU NL SE

(71) Applicant: MERRELL DOW PHARMACEUTICALS INC.
2110 E. Galbraith Road
Cincinnati Ohio 45215(US)

(72) Inventor: Miller, Francis P.
336 Broadway
Loveland Ohio 45140(US)
Inventor: Kane, John M.
6813 Dearwester Drive
Cincinnati Ohio 45236(US)

(74) Representative: Macchetta, Francesco et al
Gruppo Lepetit S.p.A. Patent and Trademark
Department 34, Via Roberto Lepetit
I-21040 Gerenzano (Varese)(IT)

## (54) Process for the preparation of 5-aryl-2,4-dialkyl-3H-1,2,4-triazole-3-thiones.

(57) This invention relates to a novel process for the preparation of 5-aryl-2,4-dialkyl-3H-1,2,4-triazole-3-thiones, useful as antidepressants, through the thionation of the corresponding 5-aryl-2,4-dialkyl-3H-1,2,4-triazol-3-ones.

EP 0 280 867 A1

## PROCESS FOR THE PREPARATION OF 5-ARYL-2,4-DIALKYL-3H-1,2,4-TRIAZOLE-3-THIONES

This invention relates to a novel process for the preparation of 5-aryl-2,4-dialkyl-3H-1,2,4-triazole-3-thiones, useful as antidepressants.

More specifically, this invention relates to compounds of the formula

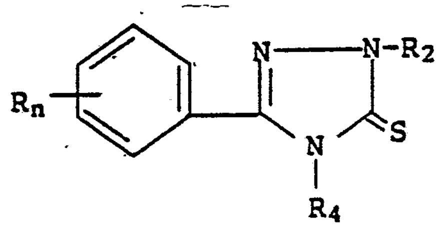

I

and the tautomers thereof, wherein

R represents halogeno, $C_{1-6}$ lower alkyl, $C_{1-6}$lower alkoxy, hydroxy, methylenedioxy or trifluoromethyl, with n being 0, 1, 2, and each of $R_2$ and $R_4$ independently represent $C_{1-6}$ lower alkyl.

For R, preferably halogeno represents chloro or fluoro, and methyl and ethyl represent the preferred lower alkyl moieties, although all the straight, branched and cyclic manifestations thereof such as n-propyl, cyclopentyl, cyclohexyl and cyclopropyl are herein included. Lower alkoxy radicals include ethers having alkyl moieties paralleling the $C_{1-6}$ alkyl group. Preferably n is one, representing a mono-substituted phenyl moiety with the R-substitutent being a group located at any of the ortho, meta or para positions, although the para-substituted compounds are preferred. When di-substituted (i.e., n is 2), the 2,3-; 2,4-; 2,5-; 2,6-; 3,4-; and 3,5-positions are contemplated. The tautomeric forms are included for each of the compounds embraced within formula I. Preferably $R_2$ or $R_4$ each represent a group selected from methyl and ethyl, but may represent any straight or branched $C_{1-6}$ alkyl group.

Using standard laboratory methodology, the pharmacological properties of these compounds and their relative potencies as antidepressants may readily be demonstrated. When compared with other agents clinically known to be useful as antidepressants, the dosage regimen may readily be ascertained by those of ordinary skill in the art.

For example, the assay testing for prevention of reserpine-induced ptosis in mice and in rats is a standard assay. In those test groups, weighed mice or rats are housed individually in wire mesh stick cages and administered test compound or vehicle. At a selected time thereafter, reserpine, prepared as a 4 mg/ml solution in dilute acetic acid, is given to rats at a dose of 4 mg/kg subcutaneously, and to mice as a 0.2 mg/ml solution in dilute acetic acid at a dose of 2 mg/kg intravenously into a tail vein. In each assay the animals are examined individually in a plexiglass cylinder 90 minutes later in the rat assay or 60 minutes after administration of reserpine to mice. Prevention or delay of ptosis is considered significant if the average closure of both eyes is less than 50% after observing for 30 seconds. The $ED_{50}$ for prevention of ptosis is defined as the dose of test compound that significantly prevents ptosis in 50% of the test animals.

In these tests imipramine has an $ED_{50}$ of 2.6 mg/kg (using a 30 minute pre-treatment time) in rats, whilst 5-(4-chlorophenyl)-2,4-dimethyl-3H-1,2,4-triazole-3-thione, one of the more potent examples of the compounds of this invention, has an $ED_{50}$ of 0.14 under the same conditions. In mice, imipramine, at a 60 minute pre-treatment time, has an $ED_{50}$ of 4.1 mg/kg whilst 5-(4-chlorophenyl)-2,4-dimethyl-3H-1,2,4-triazole-3-thione has an $ED_{50}$ of 0.27 under the same conditions.

Another assay utilized to evaluate antidepressant activity is testing for the antagonism of RO-4-1284* -induced hypothermia. (*Niemegeers, Carlos, J.E. "Antagonism of Reserpine - Like Activity", edited by S. Fielding and H. Lal, published by Futura, pg. 73-98.) In this test, groups of male mice are weighed and housed individually in wire mesh stick cages. The rectal temperature of each mouse is recorded and the test compound or vehicle is then administered. At a selected time thereafter, RO-4-1284, prepared as a 2 mg/kg solution in distilled water, is administered at a dose of 20 mg/kg i.p. Mice are then placed in a cold room (36°F) for 30 minutes, and then returned to room temperature for 30 minutes. At this time (60 minutes after RO-4-1284 administration) the rectal temperature of each mouse is again recorded. Under these conditions, RO-4-1284 causes a fall in rectal temperature of more than 6°C. The final temperatures of control groups of ten RO-4-1284-treated mice from a number of experiments are combined to form an "historic control" of 100 mice. This control is updated periodically by replacement of the oldest data. Any drug-treated animal which has final temperature (after RO-4-1284) which is greater than the mean + 2 S.D.

of the RO-4-1284 historic control is considered to exhibit significant antagonism to the hypothermic effect of RO-4-1284. The $ED_{50}$ for antagonism is defined as that dose of test compound which significantly antagonizes RO-4-1284 hypothermia in 50% of the test animals.

Using a 60 minute pretreatment time and these criteria for evaluation of effects, desipramine was found to have an $ED_{50}$ of 0.1 mg/kg i.p.; imipramine, an $ED_{50}$ of 1.8 mg/kg i.p., Catron®, an $ED_{50}$ of 0.7 mg/kg i.p., and 5-(4-chlorophenyl)-2,4-dimethyl-3H-1,2,4-triazole-3-thione an $ED_{50}$ of 0.34 mg/kg i.p.

These standard laboratory tests demonstrate that the compounds of this invention have pharmacological effects generally attributed to antidepressants and thus the compounds of this invention will elevate mood in patients suffering from depression and will have an end-use application in the treatment of patients suffering from endogenous depression, a term used interchangeably with pyschotic or involutional depression. In this use, the compound (I) will exert a relatively quick onset of action and have a prolonged duration of activity. In general, the compounds may be expected to exert their anti-depressant effects at dose levels of about 0.25-25 mg/kg of body weight per day although, of course, the degree of severity of the disease state, age of the patient and other factors determined by the attending diagnostician will influence the exact course and dosage regimen suitable for each patient. In general the parenterally administered doses are about $\frac{1}{4}$ to $\frac{1}{2}$ that of the orally administered dose.

For oral administration, the compounds can be formulated into solid or liquid preparations such as capsules, pills, tablets, troches, powders, solutions, suspensions or emulsions. The solid unit dosage forms can be a capsule which can be of the ordinary gelatin type containing, for example, lubricants and inert filler, such as lactose, sucrose or cornstarch. In another embodiment, the compounds of general formula I can be tableted with conventional tablet bases such as lactose, sucrose and cornstarch, in combination with binders, such as acacia, cornstarch or gelatin, disintegrating agents such as potato starch or alginic acid, and a lubricant such as stearic acid or magnesium stearate.

For parenteral administration, the compounds maybe administered as injectable dosages of a solution or suspension of the compound in a physiologically acceptable diluent with a pharmaceutical carrier which can be a sterile liquid such as water, alcohols, oils and other acceptable organic solvents, with or without the addition of a surfactant and other pharmaceutically acceptable adjuvants. Illustrative of oils which can be employed in these preparations are those of petroleum, animal, vegetable, or synthesic origin, for example, peanut oil, soybean oil and mineral oil. In general, water, saline, aqueous dextrose and related sugar solutions, ethanol and glycols such as propylene glycol or polyethylene glycol, or 2-pyrrolidone are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert material such as biodegradable polymers or synthetic silicones, for example Silastic®, a silicone rubber manufactured by the Dow-Corning Corporation.

As is true in many classes of compounds generally suitable for any particular pharmacological activity having a therapeutic end-use application, certain subgeneric groups and certain specific members of the class, because of their overall therapeutic index, biochemical and pharmacological profile, are preferred. In this instance the preferred compounds are those wherein both $R_2$ and $R_4$ groups are methyl or ethyl, those wherein the R substituent is chloro or fluoro, those wherein the $R_n$substituent is a monochloro or monofluoro substituent preferably located at the 4-position, and those wherein $R_n$ represents dichloro or difluoro substitution preferably at the 2,4-or 2,6 position. Specifically preferred compounds are:

5-phenyl-2,4-dimethyl-3H-1,2,4-triazole-3-thione
5-(4-chlorophenyl)-2,4-dimethyl-3H-1,2,4-triazole-3-thione
5-(4-fluorophenyl)-2,4-dimethyl-3H-1,2,4-triazole-3-thione
5-(2-fluorophenyl)-2,4-dimethyl-3H-1,2,4-triazole-3-thione
5-(2,6-difluorophenyl)-2,4-dimethyl-3H-1,2,4-triazole-3-thione
5-(3-fluorophenyl)-2,4-dimethyl-3H-1,2,4-triazole-3-thione
5-(4-methylphenyl)-2,4-dimethyl-3H-1,2,4-triazole-3-thione
5-(2,4-dichlorophenyl)-2,4-dimethyl-3H-1,2,4-triazole-3-thione
5-(2,4-difluorophenyl)-2,4-dimethyl-3H-1,2,4-triazole-3-thione
5-(3,4-difluorophenyl)-2,4-dimethyl-3H-1,2,4-triazole-3-thione

Compounds of formula I may be prepared by a novel thionation process wherein 2,4-dihydro-3H-1,2,4-triazol-3-ones may be converted to their corresponding thione derivatives. The so-obtained thiones have been prepared by other methods and are the subject matter of European Patent Application Publication No. 0221485.

Numerous thionation methods have been reported in the literature. Among these, Lawesson's reagent is considered to be the reagent of choice for the conversion of a keto moiety to a thione. Other reagents, such as $P_2S_5$ have also been used with some success. Unfortunately, the prior art procedures have not been suitable for the conversion of 2,4-dihydro-3H-1,2,4-triazol-3-ones to the thiones of this invention. Indeed, even the reagent of choice, i.e., Lawessons's reagent, works with such poor yields that its practical application to the appropriate triazol-3-ones for preparation of the compounds of this invention is not feasible.

The compounds of this invention may be prepared by the conversion of the herein described 2,4-dihydro-3H-1,2,4-triazol-3-ones by reaction with bis(tricyclohexyltin) sulfide in the presence of a boron trihalide. This reaction may be schematically depicted by the following reaction scheme.

Reaction Sdheme

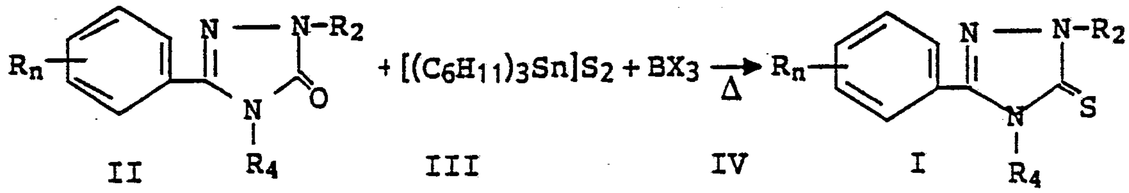

II III IV I

wherein $R_n$, $R_2$ and $R_4$ are as defined above and X represents a halogen atom, preferably chlorine.

In effecting this thionation process, it is preferred to use molar concentrations of the reactants II, III, and IV in the ratio of about 1.0: 1.5: 1.0, respectively. The reaction is effected in a suitable anhydrous solvent at about the reflux temperature of the reaction mixture, generally about 100° to 150°C, generally for a period of about 15 to 24 hours. The preferred solvent is toluene although other equivalently functioning solvents (e.g., benzene, xylene and the like) may also be employed. As anhydrous conditions give best results, sieve-dry toluene makes for an excellent solvent and running the reaction under an inert gaseous atmosphere, i.e., using a nitrogen or argon atmosphere, are preferred reaction conditions. Other boron halides may be used, but boron trichloride is preferred.

In those instances wherein $BCl_3$ is the catalyst for the reaction, the isolation of the desired thione of formula I is a problem because of the difficulty in separating the thione from the reaction by-product, presumably tricyclohexyltin chloride. This difficulty is obviated by reaction of the by-product with KF, thereby converting it to its corresponding, but insoluble, fluoride; the resulting insoluble compound being readily removed by filtration. Of course, the use of boron trifluoride as a catalyst would obviate this problem but it is still preferred to use $BCl_3$. (References related to the foregoing are: J. Am. Chem. Soc., 104, 3104 (1982); J. Org. Chem., 44, 449 (1979) and J. Org. Chem., 43, (1978).

The intermediate compounds of Formula II may readily be prepared using processes and techniques analogously known in the art as seen by the following reaction scheme:

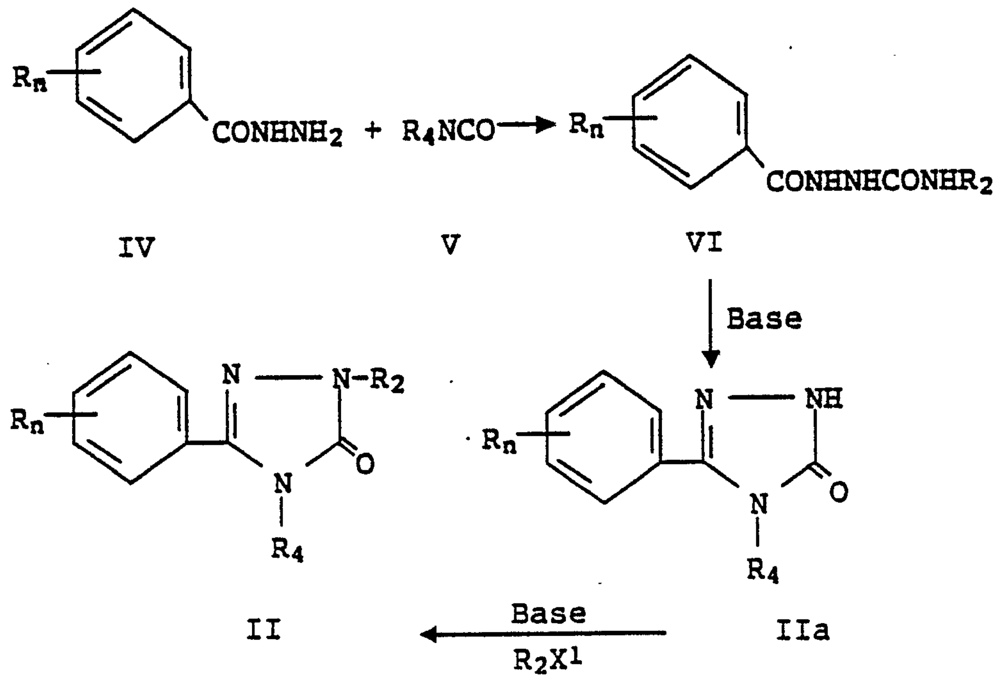


IV V VI

II IIa

wherein $R_n$, $R_2$ and $R_4$ are as defined in formula I and $X^1$ is a suitable leaving group.

The preparation of the 1-aroyl-4-substituted-semicarbazides (VI) is readily effected by heating a hydrazide (IV) with an isocyanate (V) by contacting the reactants together in a suitable aprotic solvent, preferably one in which the hydrazide reactant is soluble, e.g., tetrahydrofuran (THF), $CHCl_3$, $CH_2Cl_2$, benzene, toluene, $Et_2O$ and the like. If alcohols are used, they must be anhydrous. The reaction is quite rapid and may be carried out at 0°C to about room temperature and, although the reaction proceeds rapidly, the mixture may be left for 24 hours without any significant decrease in yield. The required hydrazides and isocyanates are readily available but may be prepared by known techniques quite obvious to one of ordinary skill in the art.

4-Substituted-5-aryl-2,4-dihydro-3H-1,2,4-triazol-3-ones (IIa) may be prepared by reacting the semicarbazides (VI) with a base, preferably an aqueous alkali metal hydroxide (e.g., NaOH, KOH) at about 50-120°C although reflux temperatures are preferred. Normal reaction time is about 7 hours although 4-24 hours may be needed depending on the temperature of the mixture.

The desired 2,4-disubstituted-2,4-dihydro-3H-1,2,4-triazol-3-ones (II) may be prepared by reacting 4-substituted-2,4-dihydro-3H-1,2,4-triazol-3-ones (IIa) with an appropriate $R_2X^1$ reactant wherein $X^1$ is a suitable leaving group, e.g., Cl, Br, $OSO_2CF_3$, and the like. Preferably the reaction takes place in a solution of an aqueous alkali metal hydroxide (e.g., KOH, NaOH), although more reactive bases (e.g., NaH, KH, LDA) may be used if the reaction is affected under aprotic dry conditions. The reaction preferably takes place at room temperature over periods of about 18 hours to two weeks.

The following specific examples are given to illustrate the preparation of the compounds of this invention although the scope of compounds exemplified is not meant to be limiting.

## Preparation of Intermediate 1-Aroyl-4-substituted semicarbazides

### EXAMPLE 1

### 1-(4-Chlorobenzoyl)-4-ethylsemicarbazide

A stirred suspension of 4-chlorobenzoic acid, hydrazide (17.1 g, 1.00 x $10^{-1}$ mole) and THF (425 ml) was warmed until homogeneous, at which time ethyl isocyanate (8.7 ml, 1.1 x $10^{-1}$ mole) was added via syringe. A precipitate soon formed. After stirring overnight the reaction was diluted with $Et_2O$ and the precipitate was collected by filtration affording a colorless powder: 23.7 g (98%). Crystallization from ethanol gave a colorless solid: 21.4 g (88%), mp 237-239°.

## Preparation of 5-Aryl-4-substituted-2,4-dihydro-3H-1,2,4-triazol-3-ones

EXAMPLE 2

5-(4-chlorophenyl)-2,4-dihydro-4-ethyl-3H-1,2,4-triazol-3-one

1-(4-chlorobenzoyl)-4-ethylsemicarbazide (23.7 g, 9.81 x $10^{-2}$ mole) and 1 molar aqueous NaOH (118 ml, 1.18 x $10^{-1}$ mole) were stirred and warmed to reflux. After refluxing 23 hours, heating was discontinued and the reaction was acidified by the dropwise addition of 1 molar aqueous hydrochloric acid (130 ml, 1.30 x $10^{-1}$ mole). A colorless solid formed as the reaction was acidified and, after cooling in an ice bath, this was collected by filtration. Crystallization from isopropanol gave colorless spars: 18.2 g (83%), mp 188-189°.

Preparation of 5-Aryl-2,4-dihydro-2,4-disubstituted-3H-1,2,4-triazol-3-ones

EXAMPLE 3

5-(4-Chlorophenyl)-2,4-dihydro-4-ethyl-2-methyl-3H-1,2,4-triazol-3-one

To a stirred, room temperature solution of 5-(4-chlorophenyl)2,4-dihydro-4-ethyl-3H-1,2,4-triazol-3-one (6.00 g, 2.68 x $10^{-2}$ mole) and 1 molar aqueous NaOH (30.0 ml, 3.00 x $10^{-2}$ mole) was added a solution of methyl iodide (2.5 ml, 4.0 x $10^{-2}$ mole) and ethanol (10 ml). After stirring overnight at room temperature, the reaction mixture was transferred to a separatory funnel where it was extracted three times with EtOAc. The EtOAc extracts were combined, washed with saturated aqueous NaCl, and dried over $Na_2SO_4$. The drying agent was removed by filtration and the filtrate was evaporated at reduced pressure leaving an oil which slowly solidified. Chromatography and crystallization from cyclohexane gave small colorless needles: 3.4 g (53%), mp 73-75°.

In a similar manner the following compounds also may be prepared.

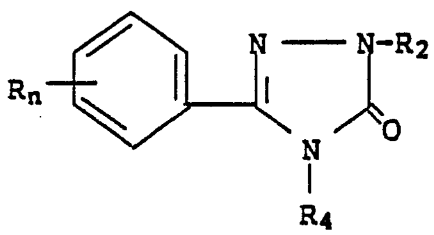

| $R_n$ | $R_2$ | $R_4$ | M.P. °C |
|---|---|---|---|
| H | $CH_3$ | $CH_3$ | 140-141 |
| H | $C_2H_5$ | $CH_3$ | 87-89 |
| H | $CH_3$ | $C_2H_5$ | oil |
| 2-Cl | $CH_3$ | $CH_3$ | 61-63 |
| 4-Cl | $CH_3$ | $CH_3$ | 126-128 |
| 4-Cl | $C_2H_5$ | $CH_3$ | 79-81 |
| 4-Cl | $CH_3$ | $C_2H_5$ | 73-75 |
| 4-Cl | $C_2H_5$ | $C_2H_5$ | 62-64 |
| 4-Cl | n-$C_3H_7$ | $C_2H_5$ | oil |
| 2-F | $CH_3$ | $CH_3$ | 69-71 |
| 4-F | $CH_3$ | $CH_3$ | 104-106 |
| 3,4-$Cl_2$ | $CH_3$ | $CH_3$ | 107-109 |
| 4-$CH_3$ | $CH_3$ | $CH_3$ | 92-94 |
| 4-$CH_3O$-3-(n-$C_4H_9O$) | $CH_3$ | $CH_3$ | 112-114 |
| 4-$CH_3O$-3-(cyclo-$C_5H_9O$) | $CH_3$ | $CH_3$ | 153-155 |

The foregoing generically depicted thionation process is illustrated by the following example.

## EXAMPLE 4

5-(4-Chlorophenyl)-2,4-dimethyl-3H-1,2,4-triazole-3-thione

To a stirred, room temperature, solution of 5-(4-chlorophenyl)-2,4-dimethyl-3H-1,2,4-triazole-3-one (0.90 g, 4.0 x $10^{-3}$ mole), bis(tricyclohexyltin) sulfide (4.63 g, 6.02 x $10^{-3}$ mole) and sieve dry toluene (63 ml), was added via syringe a methylenechloride solution of $BCl_3$ (4.1 ml, 4.1 x $10^{-3}$ mole) The reaction was then heated to reflux. After refluxing overnight, the entire reaction mixture was transferred to a 500 ml round bottom flask and the toluene was evaporated at reduced pressure. The solid concentrate was slurried with $Et_2O$ (250 ml) and 10% aqueous KF (100 ml) was added. The flask was stoppered and the two phases shaken. This results in the formation of a colorless solid, presumably $(C_6H_{11})_3SnF$, which was removed by filtration. The filtrate was transferred to a separatory funnel and the aqueous KF layer was separated. The ethereal layer was washed with sat. aqueous NaCl (100 ml) before being dried over anhydrous $Na_2SO_4$. The drying agent was removed by filtration and the filtrate was evaporated at reduced pressure leaving a yellowish oil which solidified to a pale yellow solid: 2.0 g. The oil was flash chromatographed with 2%

$EtOAc/CH_2Cl_2$ affording a colorless (faintly yellowish) solid: 0.71 g. The solid is crystallized from isopropanol to yield the desired product as colorless plates: 0.68 g (71%) m.p. 114-116°C.

In a similar manner, by substituting the reactants of examples 1-4 with appropriate $R_2$, $R_4$-substituted reactants, and by substantially following the techniques therein, the following compounds are readily prepared.

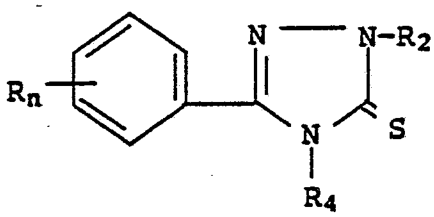

I

| $R_n$ | $R_2$ | $R_4$ | M.P. °C |
|---|---|---|---|
| 4-Cl | $CH_3$ | $C_2H_5$ | 113-115° |
| 4-F | $CH_3$ | $CH_3$ | 130-132° |
| H | $CH_3$ | $CH_3$ | 133-135° |
| H | $C_2H_5$ | $CH_3$ | 105-107° |
| 4-Cl | $C_2H_5$ | $CH_3$ | 118-120° |
| 4-Cl | $C_2H_5$ | $C_2H_5$ | 91-93° |

Other compounds embraced within formula I may similarly be prepared by using the procedures of Example 1-4.

## Claims

1. A process for the preparation of compound of the formula

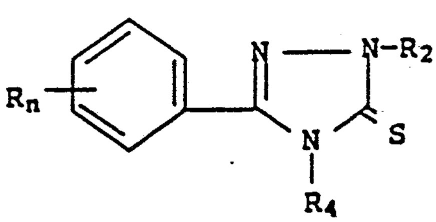

I

and the tautomers thereof, wherein

R is halogeno, $C_{1-6}$ lower alkyl, $C_{1-6}$ lower alkoxy, hydroxy, trifluoromethyl, or methylenedioxy,

n is 0, 1 or 2, and

$R_2$ and $R_4$ independently represent $C_{1-6}$ lower alkyl, which comprises thionating the corresponding 2,4-dihydro-3H-1,2,4-triazol-3-one by reacting a 2-$R_2$-4-$R_4$-5-$R_n$-phenyl-2,4-dihydro-3H-1,2,4-triazol-3-one wherein R, $R_2$ $R_4$ and n are as defined above with bis-(tricyclohexyltin)sulfide in the presence of boron trichloride.

2. A process according to claim 1 wherein the isolation of the final product is facilitated by treating the reaction mixture with potassium fluoride.

3. A process of claim 1 for preparing a compound of formula I wherein R is chloro.

4. A process of claim 1 for preparing a compound of formula I wherein each of $R_2$ and $R_4$ is methyl.

5. A process of claim 1 for preparing a compound of formula I wherein each of $R_2$ and $R_4$ is methyl and R is chloro.

6. A process of claim 1 for preparing a compound of formula I wherein each of $R_2$ and $R_4$ is methyl and R is fluoro.

7. A process of claim 1 for preparing a compound of formula I wherein n is one.

8. A process of claim 1 for preparing a compound of formula I wherein each of $R_2$ and $R_4$ is methyl, R is chloro and n is one.

9. A process of claim 1 for the preparation of 5-(4-chlorophenyl)-2,4-dimethyl-3H-1,2,4-triazole-3-thione.

European Patent Office

# EUROPEAN SEARCH REPORT

Application Number

EP 88 10 1024

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | JOURNAL OF THE AMERICAN CHEMICAL SOCIETY, vol. 104, no. 11. 2nd June 1982, pages 3104-3106; K. STELIOU et al.: "Tin-assisted sulfuration: A highly potent new method for the conversion of carbonyl units into their corresponding thiocarbonyl analogues" * whole document * | 1 | C 07 D 249/12 |
| A,D | JOURNAL OF ORGANIC CHEMISTRY, vol. 44, no. 3, 2nd February 1979, page 449; J.E. LEIBNER et al.: "Facile product isolation from organo-stannane reductions of organic halides" * whole document * | 2 | |
| A | ACTA CHEMICA SCANDINAVICA, vol. B38, no. 5, 1984, paGes 397-405; I. PETTERSON et al.: "Conformational analysis of crowded anilides and thioanilides. The "en face" steric effort of aryl groups" * page 398 * | 1 | **TECHNICAL FIELDS SEARCHED (Int. Cl.4)** |
| P,A D | EP-A-0 221 485 (MERRELL DOW PHARMACEUTICALS INC.) * claims 1-8, 11 * | 1,3-9 | C 07 D 249/00 |

The present search report has been drawn up for all claims

| Place of search | Date of completion of the search | Examiner |
|---|---|---|
| BERLIN | 16-05-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)